# EUROPEAN PATENT APPLICATION

(11) **EP 2 082 751 A2**
(43) Date of publication of application: **29.07.2009**
(21) Application number: 09000901.0
(22) Date of filing: 23.01.2009
(51) Int. Cl.: A61K 47/02, A61K 47/36, A61K 33/00

(54) **Topical formulations in the form of aqueous gels fro the treatment of disorders of the skin and mucous membranes or as vehicules for transdermal administration of natural extractive preparations, substances, molecules and drugs**

(30) Priority: 25.01.2008 IT MI20080114
(71) Applicant: Ceresa, Gian Battista, 23030 Livigno (SO) (IT); Sala Peup, Anna Maria, 23030 Livigno (SO) (IT)
(72) Inventor: Ceresa, Gian, Battista, 23030 Livigno (IT)
(74) Representative: Minoja, Fabrizio

(57) **Abstract**

Disclosed are topical formulations in the form of aqueous gels comprising hydrolysed water and one or more gelling agents and possibly one or more drugs, molecules and natural extractive substances, in particular an analgesic, an antihistamine and carotenoids of natural extraction.

## Description

The present invention relates to topical formulations in the form of aqueous gels comprising hydrolysed water, one or more gelling agents and possibly one or more drugs, molecules and natural extractive substances, in particular an analgesic, an antihistamine and carotenoids of natural extraction.

### Prior art

Among the forms designed for topical administration, aqueous gels are often preferred, if they are compatible with the proposed applications, because they are generally more convenient to use than formulations with a fatty base such as ointments. Moreover, not all molecules, drugs and substances can be formulated as aqueous gels due to solubility, stability and/or bioavailability problems. New systems and processes which can eliminate these drawbacks are therefore constantly being sought.

Recently, a number of studies were published on the properties of different forms of water characterised by molecular aggregates or clusters of different sizes. WO 2007/048772, filed by Akuatech, describes the electrolytic preparation of acidic water with cosmetic and disinfectant properties.

### Description of the invention

It has now been found that aqueous gels useful as such for the treatment of disorders of the skin and mucous membranes, or as promoters of absorption of molecules, drugs and substances which otherwise present little or no absorption by topical administration, can be obtained by subjecting the water to an electrolytic process followed by the addition of gelling agents and possibly other ingredients that stabilise the clusters of water molecules and the ion products deriving from electrolysis in a configuration that preserves new and advantageous properties.

### Detailed description of the invention

The hydrolysed water of the invention is obtained by hydrolysis in a electrolyser fitted with a semipermeable membrane which separates the anode and cathode: the acidic water in the anode compartment and the alkaline water in the cathode compartment are combined at the end of the electrolysis process until the desired pH value is obtained: generally between 2.5 and 9.0. depending on the application. A substantially neutral pH is preferred, in particular between 6.5 and 7.5, and preferably pH 7.0. The electrolysis unit can be the conventional type with platinum-covered titanium mesh electrodes of suitable dimensions in relation to the volume of the electrolysis chamber. The membrane is available on the market, for example from Dupont, under the tradename Nafion®.

The water to be hydrolysed can be distilled water to which a sodium, potassium or lithium salt is added, typically sodium, potassium or lithium chloride or sulphate, in amounts ranging from 0.1 to 6 g/l, preferably 1.5 to 4 g/l, or a natural spring water, mineral water or water with a low mineral content.

Any chlorine developed as a result of hydrolysis is removed by stirring, stripping, chemical reaction or filtration through activated carbon.

At least one gelling agent is then added to the water thus treated, such as carrageenans obtained from *Chondrus crispus* and *Gigartina mamitiosa* seaweed, agar agar, xanthan gum from *Xanthomonas campestris,* guar, pectins, gelatins, alginates, starches, cellulose or derivatives thereof, or other natural or synthetic polymers or polysaccharides which are conventionally used to increase the density of aqueous solutions. The preferred gelling agents are xanthan gum or carrageenans, which are available on the market from several manufacturers. The amount of gelling agent can vary within a wide range, and will depend on the desired consistency of the gel and the type of gelling agent selected; however, broadly speaking it will be between 0.2 and 3% by weight, preferably between 0.25 and 1.9%.

The gelling agent can also be added directly to the alkaline water or acidic water collected from the cathode or anode chamber, before they are mixed. In that case, alkaline gels or acid gels as well as neutral gels can be obtained.

The invention therefore also relates to a process for the preparation of the formulations according to the invention, which comprises:
a) hydrolysis of water in an electrolyser equipped with a semipermeable membrane which separates the anode and cathode;
b) collection and mixing of the acid and alkaline water from the anode and cathode compartments;
c) addition of gelling agents and possibly preservatives and other ingredients of the compositions, preferably at a temperature of between 50 and 65°C.

Electrolytic treatment is believed to alter the natural arrangement of the water molecules. The gelling agent appears to stabilise that configuration and the ionic products of electrolysis, which are also believed to be responsible for the advantageous, unexpected properties of the formulations according to the invention. The existence of these different chemical forms has formed the subject of various studies, and can be demonstrated by nuclear magnetic resonance spectroscopy or chemical identification of the ions produced and present. In any event, the validity of the invention is in no way dependent on the correctness of this theory.

The formulations of the invention are preferably completed by the addition of preservatives, such as parabens or sodium benzoate, glycerol, essential oils, flavourings, perfumes, hydroxyacids, emollients, soothing ointments and possibly other excipients in conventional use for the formulation of compositions with an aqueous base for topical use.

The gels of the invention can be used as such for the treatment of disorders of the skin and mucous membranes without the addition of drugs, or as vehicles for transdermal administration of molecules, drugs and natural extractive substances which present little or no absorption by this administration route. A preferred example of said drugs is paracetamol, one of the most potent analgesics, which is currently available only for enteral administration, because its chemico-physical properties prevent a topical and transdermal formulation, unlike other painkillers and non-steroidal antiinflammatory drugs such as diclofenac and ibuprofen. This invention makes topical/transdermal administration of paracetamol possible, and provides considerable advantages in terms of therapeutic efficacy and tolerability, not only by comparison with oral administration of the drug, which has some potentially toxic side effects, but also by comparison with the known topical formulations of other drugs, which require (*inter alia)* complex technologies (transdermal patches) and/or absorption promoters or other expensive vehicles which are not always well tolerated. Another molecule which is made absorbable by the gel is sodium cromoglycate, an antihistamine, which thus becomes available for topical administration, for example in the treatment of mastocytosis. Carotenoids extracted from fresh carrots can be transported by the gel through the skin to the deep tissues and the vessels of the arterial, venous and lymphatic circulatory system.

A preferred aspect of the invention is therefore a formulation in the form of an aqueous gel for topical use containing paracetamol as active ingredient and a vehicle consisting of hydrolysed gelled water, as described above. The percentage of paracetamol can range from 0.5% to 3% by weight, and preferably from 1 to 1.5%.

The gel according to the invention enables paracetamol to be absorbed in effective doses through the skin and the deep tissues below it, thus allowing the drug to reach the muscle bands, tendons, nerves, muscles, cartilage and joints, and perform its powerful analgesic action.

The paracetamol gel forming the subject of the invention was administered to patients suffering from acute disorders of the musculoskeletal system (muscle spasms and pains caused by effort, pulled muscles, torn muscles and tendons, sprained joints, sequelae of dislocated joints and bone fractures, tendinitis, fasciitis, radiculitis, scapulo-humeral periarthritis, epicondylitis and epitrochleitis of the elbow, lower back pain, temporal arthritis) and chronic pains (joint pains caused by osteoarthritis, osteoarticular pain caused by osteoporosis, and chronic primary rheumatoid arthritis). In the case of acute forms, 270 patients were treated for an average period ranging between 4 and 14 days, often with a complete response (100% of the patients treated for each disorder), and with no reactions of skin irritation, intolerance or allergy. The gel was rubbed gently into the painful area until it was completely absorbed (2-3 mins.). The treatment can be repeated 4 or 5 times in a 24-hour period. In the case of long-term treatment of chronic forms (58 patients), especially pain caused by osteoarthritis (18 patients) and chronic primary rheumatoid arthritis (32 patients), a very good clinical action was again observed, with a favourable response in 88% and 95% of patients treated respectively. Above all, the response was much faster than with other topical drugs in current in use (gels, creams, ointments and medicated patches), and compliance was excellent; in the majority of cases, patients obtained immediate benefits from the administration of the gel which, they said, "makes you forget the pain".

According to a further preferred aspect, the gel according to the invention, as such, can be advantageously used for the treatment of skin disorders such as atopic dermatitis, all forms of common acne, acne rosacea, first and second degree burns, skin irritations, insect bites, itchy states of the skin and mucous membranes, and bacterial, inflammatory and painful disorders of the teeth and mucous membranes of the mouth, including bacterial plaque. In particular in atopic dermatitis, a mainly paediatric disorder which the available treatments (topical corticosteroids, emollients, antihistamines and, more recently, immunosuppressants such as Tacrolimus and Pimecrolimus) are as yet unable to cure, the application of the gel obtainable from water previously electrolysed and gelled, with the addition of preservatives alone and possibly essential oils, perfumes and/or emollients, leads to a surprising, rapid reduction and eventual disappearance of the typical symptoms of this disorder, especially erythema, exudations and scab-forming lesions.

The mouthwash, due to its disintegrating and bactericidal power, acts on dental bacterial plaque, which is eliminated or greatly reduced. This formulation is effective in the tooth crevices as a "non-toothpaste". A considerable advantage of the gel according to the invention is that it does not damage the skin trophism, even if the applications are repeated and prolonged, unlike products containing steroids of natural or synthetic origin.

The invention will be illustrated in greater detail in the following Examples.

### Example 1. Preparation of a gel containing 1 - 1.5% paracetamol

1. The alkaline water and acidic water are collected at the cathode and anode respectively after electrolysis of distilled water with the addition of 4 g/litre of sodium sulphate.
2. The acid and alkaline waters are mixed until pH 7 is obtained.
3. The following preservatives and ingredients are added and dissolved in water pre-heated to 60-65°C.

| | |
|---|---|
| Paramethyl | 0.24% |
| Sodium benzoate | 0.15% |
| Paracetamol | 1-1.5% |
| Glycerin | 6% |
| Resassol vs® | 0.036% |
| Pine essence | 0.036% |
| Citric acid | 0.2% |
| Getralacta I (gelling agent) | 1.5% |

### Example 2. Preparation of a gel for atopic dermatitis

1. The acidic water and alkaline water are collected at the cathode after electrolysis of distilled water with the addition of 4 g/litre of sodium sulphate.
2. The acidic water is mixed with the alkaline water to pH 3.
3. The following preservatives and ingredients are added and dissolved in water pre-heated to 60-65°C.

| | |
|---|---|
| Paramethyl | 0.22% |
| Citric acid | 0.3% |
| Glycerin | 6% |
| Getralacta I (gelling agent) | 0.3-1.5% |

### Example 3. Preparation of a gel to soothe toothache

1. The acidic water is collected at the anode and the alkaline water at the cathode after electrolysis of distilled water with the addition of 4 g/litre of sodium sulphate.
2. The acidic water is mixed with the alkaline water to pH 3.
3. The following preservatives and ingredients are added and dissolved in water pre-heated to 60-65°C.

| | |
|---|---|
| Paramethyl | 0.2% |
| Ascorbic acid | 0.1% |
| Paracetamol | 1-1.5% |
| Eugenol | 10 drops |
| Getralacta I (gelling agent) | 1.5% |

### Example 4. Preparation of a mouthwash gel

1 The alkaline water and acidic water are collected from the cathode and anode compartments of the electrolyser after electrolysis of a 3.5% aqueous solution of sodium sulphate.
2. The waters are mixed until pH 3 is obtained.
3. The following preservatives and ingredients are added and dissolved at a temperature of 60-65°C.

| | |
|---|---|
| Paramethyl | 0.2% |
| Crystalline citric acid monohydrate | 0.2% |
| Disodium monofluorophosphate | 0.7% |
| Disodium fluoride | 0.03% |
| Peppermint essence | 8 drops/litre |
| Getralacta I (gelling agent) | 0.3% |

### Example 5. Preparation of a gel containing carotenoid oil.

1. Carotenoids are extracted mechanically from fresh carrots.
2. The juice obtained is treated immediately with 1.85% g of ascorbic acid.
3. The light phase and heavy phase of the squeezed juice are collected after decanting, and 0.1% salicylic acid is added.
4. Reasassol VS® 3.8% is added.
5. Gel is added in the proportion of 8:1 in volume to the paracetamol prepared as described in example no.1, with dissolved:

| | |
|---|---|
| Resassol vs® | 0.03%. |
| Scots pine essence | 0.03%. |
| Getralacta I (gelling agent) | 1.8-2%. |

## Claims

1. Topical formulations in the form of aqueous gels comprising hydrolysed water and one or more gelling agents.

2. Formulations as claimed in claim 1, wherein the gelling agent is selected from carrageenans obtained from *Chondrus Crispus* and *Gigartina mamitiosa* seaweed, agar agar, xanthan gum from *Xanthomonas campestris,* guar, pectins, gelatins, alginates, amides or cellulose or derivatives thereof.

3. Formulations as claimed in claim 2, wherein the gelling agent is carrageenans obtained from *Chondrus Crispus* and *Gigartina mamitiosa* seaweed, or xanthan gum from *Xanthomonas campestris.*

4. Formulations as claimed in claim 1 or 2, wherein the gelling agent is present in percentages between 0.2 and 3% by weight, preferably between 0.25 and 1.5%.

5. Formulations as claimed in any one of the preceding claims, having a pH between 2.5 and 9.

6. Formulations as claimed in any one of the preceding claims, further containing preservatives.

7. Formulations as claimed in any one of the preceding claims, further containing one or more analgesics and/or anti-inflammatories, antihistamines and carotenoids.

8. Formulations as claimed in claim 7, wherein the drug is paracetamol.

9. Formulations as claimed in any one of the preceding claims, wherein the aqueous phase is **characterised by** the presence of clusters of 5-6 water molecules with ions deriving from the process of electrolysis of the saline solutions of the anode and cathode chamber.

10. Use of the formulations as claimed in claims 1-6 for the preparation of a medicament, device or aid for the treatment of atopic dermatitis and mastocytosis.

11. Use of the formulations claimed in claims 7 and 8 for the preparation of a medicament for the treatment of acute and chronic disorders of the musculoskeletal system and disorders of the venous, arterial and lymphatic circulatory system.

12. Use of the formulations claimed in claims 1-6 for the preparation of a medicament, device or aid for oral and dental hygiene and the elimination of dental bacterial plaque, in the form of a mouthwash or "non-toothpaste".

13. Process for the preparation of the formulations as claimed in claims 1-9, comprising:
a) hydrolysis of water in an electrolyser equipped with a semipermeable membrane which separates the anode and cathode;
b) collection and mixing of the acid and alkaline water from the anode and cathode compartments;
c) addition of gelling agents and possibly preservatives and other ingredients of the compositions.

14. Process as claimed in claim 13, further including a chlorine removal step.

15. Process as claimed in claim 13 or 14, wherein the acid and alkaline water are mixed in such amounts as to obtain a pH of 2.5 to 9.

16. Process as claimed in claims 13-15, wherein step c) is conducted at a temperature of 55°-65°C.

17. Gels obtainable by the process claimed in claims 13-16.
